Europäisches Patentamt

European Patent Office

Office européen des brevets

(19)

(11) Veröffentlichungsnummer: **0 161 552**
**B1**

(12) **EUROPÄISCHE PATENTSCHRIFT**

(45) Veröffentlichungstag der Patentschrift: **25.07.90**

(21) Anmeldenummer: **85105009.6**

(22) Anmeldetag: **25.04.85**

(51) Int. Cl.⁵: **A 01 J 7/00**, F 17 D 3/10,
G 01 N 1/10

(54) **Verfahren zum Erstellen einer repräsentativen Milchprobe aus einem grösseren Milchvolumen und Probeentnahmevorrichtung.**

(30) Priorität: **10.05.84 DE 3417286**
**30.10.84 DE 3439663**

(43) Veröffentlichungstag der Anmeldung:
**21.11.85 Patentblatt 85/47**

(45) Bekanntmachung des Hinweises auf die
Patenterteilung:
**25.07.90 Patentblatt 90/30**

(84) Benannte Vertragsstaaten:
**AT BE CH DE FR GB IT LI LU NL SE**

(56) Entgegenhaltungen:
**EP-A-0 098 966**
**DE-A-1 773 656**
**DE-B-2 245 487**

(73) Patentinhaber: **Jansky, Walter**
**Kapellenweg 11**
**D-4407 Emsdetten (DE)**
(73) Patentinhaber: **Jansky, Manfred**
**Taubenstrasse 37**
**D-4407 Emsdetten (DE)**

(72) Erfinder: **Jansky, Walter**
**Kapellenweg 11**
**D-4407 Emsdetten (DE)**
Erfinder: **Jansky, Manfred**
**Taubenstrasse 37**
**D-4407 Emsdetten (DE)**

(74) Vertreter: **Patentanwaltsbüro Cohausz & Florack**
**Schumannstrasse 97**
**D-4000 Düsseldorf 1 (DE)**

**Beschreibung**

Die Erfindung bezieht sich auf ein Verfahren zum Erstellen einer für ein Milchvolumen repräsentativen Milchprobe, bei dem während des Umfüllens der Milch von einem ersten in einen zweiten Behälter ein mengenproportionaler Teilstrom aus dem Hauptstrom vom ersten Behälter in den zweiten Behälter abgezweigt und gesammelt wird und am Ende des Umfüllens der gesammelten Milch ein Teilvolumen als Probe entnommen wird.

Es ist üblich, mit Milchsammelwagen Milch unterschiedlicher Volumina und unterschiedlicher Qualität in Empfang zu nehmen. Die Milchsammelwagen sind deshalb mit einem Volumenmesser und einer Probeentnahmevorrichtung ausgerüstet, die es ermöglicht, mehr oder weniger exakt eine repräsentative Probe für die vom jeweiligen Produktionsbetrieb abzuliefernde Milch zu erstellen. Dies geschieht in der weise, daß während des gesamten Annahmevorganges ein Teilstrom aus dem Hauptstrom abgezweigt und in einen kleinen Sammel- und Mischbehälter gefüllt wird, aus dem am Ende des Annahmevorganges ein noch kleineres Volumen als Milchprobe entnommen wird. Nach Erstellen der Probe wird die übrige im Sammel- und Mischbehälter verbleibende Milch abgelassen und der bereits angenommenen Milch zugegeben. Beim nächsten Produzenten wird in gleicher weise verfahren, also der Sammel- und Mischbehälter während des Annahmevorganges mengenproportional mit Milch gefüllt. Da von der letzten Annahme im Behälter eine Benetzung geblieben ist, wird diese neue in den Sammel- und Mischbehälter gefüllte Milch mit der die Behälterwandung benetzenden Milch aus der letzten Annahme vermischt. wenn auch dieses Volumen klein ist, kann es zu einer beachtlichen Verfälschung der beim jeweils folgenden Produzenten angenommenen Milchprobe führen. Diese Gefahr der Probenverfälschung ist um so größer, je kleiner das Volumen der in den Sammel- und Mischbehälter eingefüllten Milchmenge ist. Um diese Verfälschung möglichst klein zu halten ist es bekannt (DE 22 45 487 B2) das Abzweigventil unter Berücksichtigung des jeweiligen abzunehmenden Milchvolumens auf einen großen oder kleinen Proportionalitätsfaktor einzustellen. Obgleich bei diesem Stand der Technik zum Zwecke der Beschleunigung der Erstellung der Probe eine einen Niveaustandsfühler im Sammel- und Mischbehälter aufweisende Steuereinrichtung für verschiedene Ventile vorgesehen ist, über die Probemenge in ein Probegefäß abgefüllt und der Sammel- und Mischbehälter entleert werden, ist die Erstellung der Probe nach wie vor ungenau, denn selbst eine erfahrene Bedienungsperson wird beim Abschätzen des abzunehmenden Milchvolumens kaum jedesmal das Teilungsverhältnis am Abzweigventil so einstellen können, daß der Sammel- und Mischbehälter unter optimaler Ausnutzung seiner Kapazität während des gesamten Annahmevorganges mit abgezweigter Milch gefüllt wird, ohne daß er am Ende des Annahmevorganges überfüllt wird.

Ausgehend von dieser Problematik liegt der Erfindung die Aufgabe zugrunde, ein Verfahren und eine Vorrichtung zu schaffen, mit der auf einfache Art und Weise für unterschiedlich große, von einem Produzenten anzunehmende Milchvolumina eine sehr exakte repräsentative Milchprobe erstellt werden kann.

Diese Aufgabe wird verfahrensmäßig dadurch gelöst, daß das Abzweigen der Milch in zwei Phasen mit verschiedenen Proportionalitätsfaktoren erfolgt, wobei in der ersten Phase der Proportionalitätsfaktor, bezogen auf das insgesamt umzufüllende Milchvolumen, so groß ist, daß am Ende dieser Phase ein vorgegebenes, erstes Teilvolumen erreicht ist, das dann bis auf ein einem wesentlich kleineren Proportionalitätsfaktor entsprechenden Restvolumen ausgeschieden wird, und daß in der anschließenden zweiten Phase die Abzweigung des Teilstroms mit diesem wesentlich kleineren Proportionalitätsfaktor und die Entnahme der Probe nach Vermischen des Restvolumens und des in der zweiten Phase abgezweigten Teilvolumens erfolgt.

Mit dem erfindungsgemäßen Verfahren wird eine Verfälschung der zu erstellenden Probe durch Reste von Milch in einem Sammelbehälter aus dem vorhergehenden Annahmevorgang dadurch klein gehalten, daß in der ersten phase der Sammel- und Mischbehälter gespült wird, weil das in der ersten Phase abgezweigte Milchvolumen ausreichend groß gewählt werden kann, um den Milch- und Sammelbehälter möglichst weitgehend zu füllen. Damit aber auch aus der in der zweiten Phase anfallenden Milch ein mengenproportionaler Teilstrom abgezweigt und gesammelt werden kann, wird der Behälter teilentleert. Durch Übereinstimmung der Proportonalitätsfaktoren des im Behälter verbliebenden Restvolumens und des in der zweiten Phase abgezweigten Volumens ist gewährleistet, daß am Ende des Annahmevorganges für die gesamte angenommene Milch ein repräsentatives kleines Volumen vorliegt. Dieses kleine Volumen braucht den Sammel- und Mischbehälter nicht zu füllen, da in der ersten Phase der Behälter bereits gefüllt und gespült worden ist. Während der für die ersten Phase einzustellende Proportionalitätsfaktor so gewählt werden sollte, daß bei der kleinsten anzunehmenden Milchmenge der Sammelbehälter weitgehendst gefüllt wird, wird der Proportionalitätsfaktor für die zweite Phase so gewählt, daß die Kapazität des Sammelbehälters ausreicht, um von der in der zweiten Phase anzunehmenden Milch bis zum Ende des Annahmevorganges ein Teilstrom in den Behälter gefüllt werden kann. Verfälschungen der endlich erstellten Probe durch aus dem vorhergehenden Annahmevorgang in den Leitungen und im Sammelbehälter verbliebenen Milchreste werden vernachlässigbar klein gehalten, weil in der ersten Phase das Sammelgefäß möglichst weitgehend gefüllt wird, so daß die Konzentration in dem in der ersten Phase abgezweigten Milchvolumen bereits klein ist. Es wird dann noch kleiner, weil nur die Restmenge dieses Teilvolumens bei der endgültigen Erstellung der Probe in der zweiten Phase verwendet wird.

Das erfindungsgemäße Verfahren läßt verschiedene Möglichkeiten der Ausführung zu. Nach einer ersten Ausführung wird zwischen den beiden Phasen des Umfüllvorganges eine Pause eingeschaltet, in der das in der ersten Phase abgezweigte Teilvolumen bis auf das Restvolumen ausgeschieden wird. Nach einer zweiten Ausführung ist es möglich, den Umfüllvorgang nicht zu unterbrechen. Bei diesem Verfahren wird während des Ausscheidens der aus dem ersten Teilvolumen nicht benötigten Milch die mit dem kleineren Proportionalitätsfaktor zu Beginn der zweiten Phase anfallende Milch separat gesammelt und dem Restvolumen vor Entnahme der Probe zugegeben.

Die Erfindung bezieht sich ferner auf eine Probeentnahmevorrichtung an einer Milchsammelanlage mit einer zu einem Behälter führenden Hauptleitung, von der eine mit einem auf verschiedene Teilungsverhältnisse einstellbaren Ventil ausgehenden Abzweigleitung zu einem Sammelbehälter für Proben führt, der einen Auslaß zum Abfüllen einer Probe, eine über ein Ventil gesteuerte Rücklaufleitung und einen Niveaustandsfühler einer Steuereinrichtung aufweist, mit der über Ventile die Probeentnahme steuerbar ist. Bei einer solchen Probeentnahmevorrichtung wird die Aufgabe dadurch gelöst, daß die Steuereinrichtung bei Erreichen eines vom Niveaustandsfühler festgestellten, vorbestimmten Milchniveaus das auf verschiedene Teilungsverhältnisse einstellbare Ventil von einem großen Teilungsverhältnis auf ein kleines Teilungsverhältnis umstellt und die weitere Zufuhr von mit kleinem Teilungsverhältnis abgezweigter Milch in den Sammelbehälter solange unterbricht, bis der Sammelbehälter über die ventilgesteuerte Rücklaufleitung bis auf ein dem kleinen Teilungsverhältnis entsprechendes Restvolumen entleert ist.

Um von in der ersten phase gesammelten Milch ein Restvolumen zu erhalten, das in einem bestimmten Teilungsverhältnis zu der in der ersten Phase angenommenen Milch steht, sieht eine Ausgestaltung der Erfindung vor, daß der eine Sammelbehälter zum Ausscheiden der für die Probe nicht benötigten Milch der ersten Teilmenge einen durch ein Ventil verschließbaren Überlauf ha. Dabei kann nach einer ersten Ausgestaltung der Überlauf von einer am Behälter angeschlossenen und durch ein Ventil versperrbaren Abzweigleitung gebildet sein. Nach einer alternativen Ausgestaltung kann der Überlauf von einem Rohrstück gebildet sein, das mit seiner unteren Öffnung auf den Hauptauslaß des Sammelbehälters aufsetzbar ist. Die zweite alternative Ausgestaltung hat den Vorteil, daß nur ein Ventil, und zwar das dem Hauptauslaß zugeordnete Ventil, benötigt wird.

Nach weiteren Ausgestaltungen weist die Probeentnahmevorrichtung einen weiteren Sammelbehälter auf, in den die Milch nach Umschalten des Ventils förderbar ist und dessen Inhalt nach Teilentleerung des ersten Sammelbehälters in diesen überführbar ist. Dieser weitere Sammelbehälter kann in dem ersten Sammelbehälter untergebracht sein. Mit diesen Ausgestaltungen ist es möglich, ohne Unterbrechung des Annahmevorganges mengenproportional die Milch abzuzweigen und zu sammeln.

Die weitere Förderung der abgezweigten Milch in den weiteren Sammelbehälter läßt sich vorrichtungsmäßig auf einfache Weise dadurch erreichen, daß von dem Ventil eine weitere, zu dem weiteren Sammelbehälter führende Leitung ausgeht, auf die das Ventil umschaltbar ist.

Im folgenden wird die Erfindung anhand einer zeichnung näher erläutert, die schematisch zwei Ausführungen einer Probeentnahmevorrichtung mit einem Teil einer Milchannahmeanlage zeigt. Im einzelnen zeigen

Fig. 1 eine Probeentnahmevorrichtung für eiren kontinuierlichen Annahmevorgang und

Fig. 2 eine Probeentnahmevorrichtung für einen diskontinuierlichen Annahmevorgang.

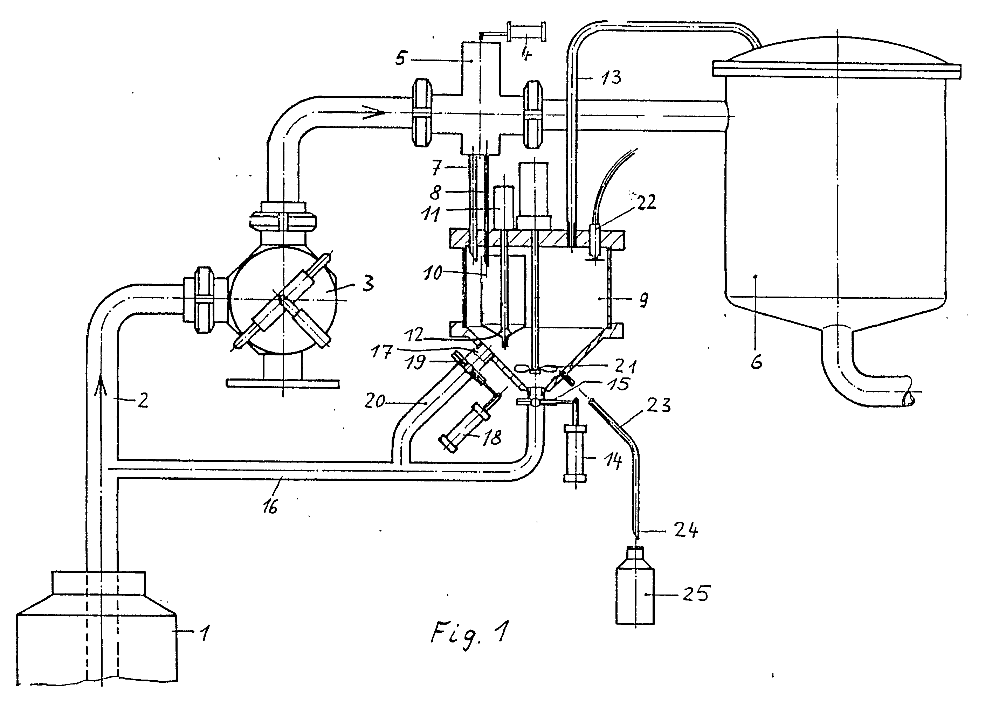

Gemäß dem Ausführungsbeispiel der Fig. 1 wird die von einem Produzenten abzuliefernde Milch in Behältern 1 zum Abholen durch einen Milchsammelwagen bereitgestellt. Über eine Hauptleitung 2, eine selbstansaugende Pumpe 3 und ein mittels einer Servobetätigung 4 auf zwei unterschiedliche Teilungsverhältnisse einstellbares Ventil 5 wird die Milch in einen Behälter 6 gefördert, aus dem die Milch in einen nicht dargestellten Sammeltank des ebenfalls nicht dargestellten Tankfahrzeuges gelangt.

Von dem Abzweigventil 5 führen zwei Abzweigleitungen 7, 8 in einen Sammel- und Mischbehälter 9, wobei die einem großen Teilungsverhältnis zugeordnete Abzweigleitung 7 unmittelbar in den Behälter 9 und die einem kleinen Teilungsverhältnis zugeordnete Abzweigleitung 8 in einen in dem Behälter 9 untergebrachten kleineren Behälter 10 mündet. Der Inhalt des kleineren Behälters 10 ist durch ein mit einer Servobetätigung 11 ausgestattetes Ventil 12 am Boden des Behälters 10 in den Behälter 9 entleerbar. Der Behälter 9 ist über eine Leitung 16 in den Behälter 6 entleerbar.

Der Inhalt des Behälters 9 kann über ein mit einer Servobetätigung 14 betätigbares Ventil 15 abgelassen und über eine Rücklaufleitung 16 in die Hauptleitung 2 eingespeist werden. Im unteren Bereich des Behälters 9 ist ein Überlauf 17 mit einem durch eine Servobetätigung 18 betätigbaren Ventil 19 und einer Verbindungsleitung 20 vorgesehen, über das der Inhalt des Behälters 9 bis auf ein Restvolumen ablaßbar und über die Leitung 16 in die Hauptleitung 2 einspeisbar ist. Mit einem in dem Behälter 9 angeordneten Rührwerk 21 kann die Milch im Behälter 9 gemischt werden. Zur Aktivierung der verschiedenen Servobetätigungen, 4, 11, 14, 18 dient eine nicht dargestellte Steuereinrichtung, die von einem Niveaustandsfühler 22 ein Signal erhält, sobald der Milchpegel im Behälter 9 den Fühler 22 erreicht.

Zur Entnahme einer Milchprobe ist am unteren Bereich des Behäters eine Leitung 23 angeschlossen, die mit einer Hohlnadel 24 in ein Probegefäß 25 einführbar ist. Damit in das Probegefäß 25 Milch gelangen kann, ist z.B. eine weitere, nicht dargestellte Hohlnadel vorgesehen, die mit der Unterdruckseite der Pumpe 3 verbunden ist. Sobald beide Nadeln 24 einen abdichtenden Dekkel des Probegefäßes 25 durchstoßen haben, gelangt die zu entnehmende Milchprobe aufgrund des Unterdruckes in das Gefäß 25.

Die beschriebene Probeentnahmevorrichtung arbeitet auf folgende Art und weise:

Nach jedem Annahmevorgang und Entnahme der Probe wird das Ventil 15 geöffnet, so daß aus dem Behälter 9 die gesamte Milch fließen kann. An der Behälterwandung bleibt nur eine Benetzung übrig. Zur Übernahme neuer Milch wird die Hauptleitung 2 mit ihrem unten offenen Ende in den Behälter 1 gesteckt und die Pumpe 3 fördert Milch aus dem Behälter 1 in den Behälter 6. Während dieser Phase ist das Ventil 5 auf ein großes Teilungsverhältnis eingestellt. Das Teilungsverhältnis ist so groß gewählt, daß unter Berücksichtigung aller anzunehmenden Milchvolumina das abzuzweigende Milchvolumen ausreicht, um den Behälter 9 möglichst weitgehend zu füllen. Dadurch wird eine Verfälschung der abgezweigten Milch durch Milchreste aus der vorhergehenden Annahme möglichst klein gehalten. Bei der Einstellung des Ventils 5 in der ersten Phase gelange die Milch über die Abzweigleitung 7 in den Behälter 9. Sobald der Milchpegel den Niveaustandsfühler 22 erreicht, gibt dieser an die Steuereinrichtung ein Signal, und die Steuereinrichtung schaltet das Ventil 5 auf das kleine Teilungsverhältnis um, so da8 die weiter abzuzweigende Milch über die Abzweigleitung 8 in den verschlossenen Behälter 10 gelangt. Gleichzeitig wird das Ventil 19 geöffnet, so daß die Milch im Behälter 9 bis auf das Niveau des Überlaufs 17 abgelassen und über die Leitung 16 zurück in die Hauptleitung 2 eingespeist wird. Nach einer vorgebbaren Verzögerungszeit schließt das Ventil 19 wieder, das Ventil 12 des Behälters 10 wird geöffnet, so daß die zwischenzeitlich im Behälter 10 gesammelte Milch mit der Milch im Behälter 9 sich vermischt.

Da die im Behälter 9 verbliebene Milch zu der in der ersten Phase angenommenen Milch in einem Teilungsverhältnis steht, das gleich dem Teilungsverhältnis nach Umschalten des Ventils 5 ist, bleibt dieses Teilungsverhältnis während der übrigen Annahmezeit in der zweiten Phase erhalten. Da die Abzweigung der Milch in einem bestimmten Teilungsverhältnis während der gesamten Annahme erfolgt, ist auch gewährleistet, daß am Ende der Annahme die im Behälter 9 befindliche Milch in ihrer Zusammensetzung repräsentativ für die insgesamt angenommene Milch ist. Am Ende der zweiten Phase der Annahme wird das probegefäß 25 an die Leitung 23 angeschlossen und in das Probegefäß 25 die gewünschte Milchprobe gefüllt. Danach wird die gesamte im Behälter 9 befindliche Milch durch Öffnen des Ventils 15 abgelassen.

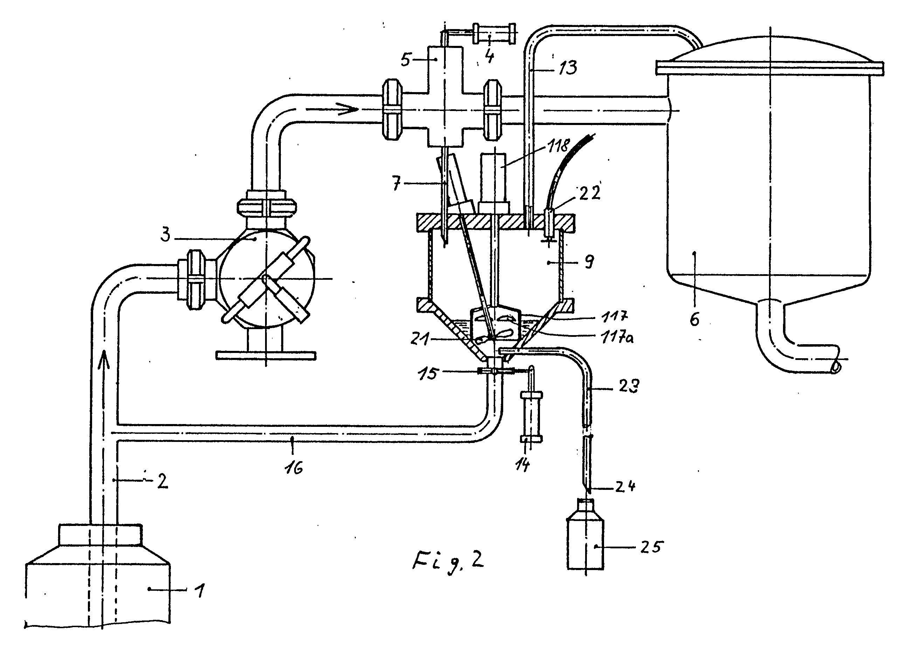

Das Ausführungsbeispiel der Fig. 2 stimmt weitgehend mit dem der Fig. 1 überein. Aus diesem Grunde tragen die gleichen Teile die gleichen Bezugszeichen. Zwischen den beiden Ausführungsbeispielen besteht der Unterschied in den Mitteln zur Erstellung des Restvolumens aus dem in der ersten Phase abgezweigten Volumen und darin, daß keine Mittel vorgesehen sind, mit denen kontinuierlicn, also auch während des Umschaltens bzw. unmittelbar nach dem Umschalten von der ersten Phase auf die zweite Phase, der Umfüllvorgang durchgeführt werden kann.

Während beim Ausführungsbeispiel der Fig. 1 der Überlauf 17 von einer vom Sammelbehälter 9 abzweigenden Leitung 20 gebildet wird, ist beim Ausführungsbeispiel der Fig. 2 im Sammelbehälter 9 ein Rohrstück 117 vorgesehen, das mit einer Servobetätigung 118 heb- und absenkbar bis vordem Hauptauslaß des Sammelbehälters 9 ist. Das Rohrstück 117 weist seitliche Überlauföffnungen 117a auf, die bei abgesenktem Rohrstück 117 das Niveau des im Sammelbehälter 9 verbleibenden Restvolumens bestimmen. Ein weiterer Unterschied zum Ausführungsbeispiel der Fig. 1 besteht darin, daß der weitere Sammelbehälter 10 und die in diesen Sammelbehälter mündende Leitung 8 fehlen.

Aufgrund dieser Unterschiede ergibt sich beim Erstellen der Probe folgender Funktionsablauf, abweichend von dem Funktionsablauf beim Ausführungsbeispiel der Fig. 1: Am Ende der ersten Phase des Umfüllvorganges wird der Umfüllvorgang gestoppt. Durch die Servobetätigung 118 wird das Rohrstück 117 dicht vor den Hauptauslaß des Sammelbehälters 9 gedrückt. Nach öffnen des Ventils 15 fließt über die Überlauföffnungen 117a die Milch bis auf ein Restvolumen ab, das als ringförmiger See zurückbleibt, wie in der Zeichnung dargestellt ist. Das Ventil 15 wird dann wieder geschlossen und der Umfüllvorgang wird fortgesetzt, wobei in der jetzigen zweiten phase Milch mit dem kleinen Proportionalitätsfaktor abgezweigt wird. Das Restvolumen der in der ersten Phase abgezweigten Milch vermischt sich dann mit dem abgezweigten Milchvolumen der zweiten Phase. Die Vermischung kann nach Anheben des Rohrstückes 117 durch das Rührwerk 21 verbessert werden.

Es versteht sich, daß bei fehlendem Gefäß 10 des Ausführungsbeispiels der Fig. 1 der Funktionsablauf der Erstellung der Probe der gleiche wie beim Ausführungsbeispiel der Fig. 2 ist. Umgekehrt läßt sich mit dem Ausführungsbeispiel der Fig. 2 bei ununterbrochenem Umfüllvorgang eine Probe erstellen, sofern im Sammelbehälter 9 ein zweiter Sammelbehälter 10, wie beim Ausführungsbeispiel der Fig. 1, angeordnet ist.

**Patentansprüche**

1. Verfahren zum Erstellen einer für ein Milchvolumen repräsentativen Milchprobe, bei dem während des Umfüllens der Milch von einem ersten in einen zweiten Behälter ein mengenproportionaler Teilstrom aus dem Hauptstrom vom ersten Behälter in den zweiten Behälter abgezweigt und gesammelt wird und am Ende des Umfüllens der gesammelten Milch ein Teilvolumen als Probe entnommen wird, dadurch gekennzeichnet, daß das Abzweigen in zwei Phasen mit verschiedenen Proportionalitätsfaktoren erfolgt, wobei in der ersten Phase der Proportionalitätsfaktor, bezogen auf das insgesamt umzufüllende Milchvolumen, so groß ist, daß am Ende dieser Phase ein vorgegebenes erstes Teilvolumen erreicht ist, das dann bis auf ein einem wesentlich kleineren Proportionalitätsfaktor entsprechenden Restvolumen ausgeschieden wird, und daß in der anschließenden, zweiten Phase die Abzweigung des Teilstroms mit diesem wesentlich kleineren Proportionalitätsfaktor und die Entnahme der Probe nach Vermischen des Restvolumens und des in der zweiten Phase abgezweigten Teilvolumens erfolgen.

2. Verfahren nach Anspruch 1, dadurch gekennzeichnet, daß zwischen beiden Phasen des Umfüllvorganges eine Pause eingelegt wird, in der das in der ersten Phase abgezweigte Teilvolumen bis auf das Restvolumen aus dem Sammelbehälter ausgeschieden wird.

3. Verfahren nach Anspruch 1, dadurch gekennzeichnet, daß bei ununterbrochenem Umfüllvorgang während des Ausscheidens der aus dem ersten Teilvolumen nicht benötigten Milch die mit dem kleineren Proportionalitätsfaktor zu Beginn der zweiten Phase anfallende Milch separat gesammelt und dem Restvolumen vor Entnahme der Probe zugegeben wird.

4. Probenahmevorrichtung an einer Milchsammelanlage mit einer zu einem Behälter (6) führenden Hauptleitung (2), von der eine mit einem auf verschiedene Teilungsverhältnisse einstellbaren Ventil (5) ausgehende Abzweigleitung (7, 8) zu einem Sammelbehälter (9) für Proben führt, der einen Auslaß (23) zum Abfüllen einer Probe, eine über ein Ventil (15) gesteuerte Rücklaufleitung und einen Niveastandsfühler (22) einer Steuereinrichtung aufweist, mit der über Ventile (5, 15, 19, 17, 117) die Probeentnahme steuerbar ist, dadurch gekennzeichnet, daß die Steuereinrichtung bei Erreichen eines vom Niveaustandsfühler (22) festgestellten vorbestimmten Milchiveaus das auf verschiedene Teilungsverhältnisse einstellbare Ventil (5) von einem großen Teilungsverhältnis auf ein kleines Teilungsverhältnis umstellt und die weitere Zufuhr von mit kleinem Teilungsverhältnis abgezweigter Milch in den Sammelbehälter (9) solange unterbricht, bis der Sammelbehälter (9) über die ventilgesteuerte Rückaufleitung (16, 20) bis auf ein dem kleinen Teilungsverhältnis entsprechendes Restvolumen entleert ist.

5. Probeentnahmevorrichtung nach Anspruch 4, dadurch gekennzeichnet, daß der Sammelbehälter (9) zum Ausscheiden der für die Probe nicht benötigten Milch des ersten Teilvolumens einen durch ein Ventil (19; 15) verschließbarem Überlauf (17; 117) hat.

6. Probeentnahmevorrichtung nach Anspruch 5, dadurch gekennzeichnet, daß der Überlauf (17) von einer am Sammelbehälter (9) angeschlossenen und durch ein Ventil (19) absperrbaren Abzweigleitung (20) gebildet ist.

7. Probeentnahmevorrichtung nach Anspruch 5, dadurch gekennzeichnet, daß der Überlauf (117) von einem Rohrstück gebildet ist, das mit seiner unteren Öffnung auf den durch ein Ventil (15) absperrbaren Hauptauslaß des Sammelbehälters (9) aufsetzbar ist.

8. Probeentnahmevorrichtung nach einem der Ansprüche 4—7, dadurch gekennzeichnet, daß ein weiterer Sammelbehälter (10) vorgesehen ist, in den die Milch nach Umschalten des Ventils (5) förderbar ist und dessen Inhalt nach Teilentleerung des ersten Sammelbehälters (9) in diesen überführbar ist.

9. Probeentnahmevorrichtung nach Anspruch 8, dadurch gekennzeichnet, daß von dem Ventil (5) (eine weitere zu dem weiteren Sammelbehälter (10) führende Abzweigleitung (8) ausgeht, auf die das Ventil (4) umschaltbar ist.

10. Probeentnahmevorrichtung Anspruch 8 oder 9, dadurch gekennzeichnet, daß der weitere Sammelbehälter (10) in dem ersten Sammelbehälter (9) untergebracht ist.

**Revendications**

1. Procédé pour prélever un échantillon de lait représentatif d'un volume de lait dans lequel, pendant le transvasement du lait d'un premier dans un second réservoir, un débit partiel du débit principal proportionnel à la quantité est dérivé du premier réservoir dans le second réservoir et collecté et, à la fin du transvasement du lait collecté, un volume partiel est prélevé comme échantillon, caractérisé en ce que la séparation du lait en deux phases s'éffectue avec des facteurs de proportionnalité différents, le facteur de proportionnalité dans la première phase, rapporté au volume de lait à transvaser au total, étant si élevé qu'à la fin de cette phase, on obtient un premier volume partiel prédéfini, qui est ensuite séparé à l'exception d'un volume résiduel correspondant à un facteur de proportionnalité beaucoup plus petit, et que, dans la seconde phase suivante, la dérivation du débit partiel avec ce facteur de proportionnalité beaucoup plus petit et le prélèvement de l'échantillon s'effectuent, après mélange du volume résiduel et du volume partiel dérivé dans la deuxième phase.

2. Procédé selon la revendication 1, caractérisé en ce qu'une pause est intercalée entre les deux phases du processus de transvasement, au cours de laquelle le volume partiel séparé dans la première phase est éliminé du récipient collecteur à l'exception d'un volume résiduel.

3. Procédé selon la revendication 1, caractérisé en ce que, dans le cas d'une opération de transvasement ininterrompue, pendant la séparation du lait non nécessaire pour le premier volume partiel, le lait produit au début de la seconde phase avec le plus petit facteur de proportionnalité est collecté séparément et ajouté au volume restant avant le prélèvement de l'échantillon.

4. Dispositif de prélèvement d'échantillon dans un dispositif collecteur de lait, avec une conduite principale (2) menant à un récipient (6) à partir duquel une conduite de dérivation (7, 8) partant d'une vanne (5) réglable selon différents rapports de division mène à un récipient collecteur (9) pour échantillons, qui présente une sortie (23) pour le soutirage d'un échantillon, une conduite de retour commandée par l'intermédiaire d'une vanne (15), et un capteur de hauteur de niveau (22) d'un dispositif de commande avec lequel, par l'intermédiaire de vannes (5, 15, 19, 17, 117) le prélèvement d'échantillon peut être commandé, caractérisé en ce que le dispositif de commande, en atteignant un niveau de lait prédéterminé mesuré par le capteur de hauteur de niveau (22) réajuste la vanne (5) réglable selon différents rapports de division depuis un rapport de division élevé jusqu'à un faible rapport de division et interrompt la continuation de l'arrivée de lait séparé avec un faible rapport de division dans le récipient collecteur (9) jusqu'à ce que le récipient collecteur (9) soit vidé, par l'intermédiaire de la conduite de retour (16, 20) commandée par vanne, à l'exception d'un volume résiduel correspondant au faible rapport de division.

5. Dispositif de prélèvement d'échantillon selon la revendication 4, caractérisé en ce que le récipient collecteur (9) présente un trop-plein pouvant être fermé par une vanne (19; 15) pour éliminer le lait de la première quantité partielle non nécessaire pour l'échantillon.

6. Dispositif de prélèvement d'échantillon selon la revendication 5, caractérisé en ce que le trop-plein (17) est constitué par une conduite de dérivation (20) raccordée au récipient (9) et pouvant être obstruée par une vanne (19).

7. Dispositif de prélèvement d'échantillon selon la revendication 5, caractérisé en ce que le trop-plein (117) est formé par une pièce tubulaire qui est démontable par son ouverture inférieure sur la sortie principale du récipient collecteur (9).

8. Dispositif de prélèvement d'échantillon selon l'une des revendications 4—7, caractérisé en ce qu'un autre récipient collecteur (10) est prévu, dans lequel le lait peut être transporté après commutation de la vanne (5) et dont le contenu peut être transmis après vidage partiel du premier récipient collecteur (9), dans celui-ci.

9. Dispositif de prélèvement d'échantillon selon la revendication 8, caractérisé en ce qu'une autre conduite (8) part de la vanne (5) et mène à l'autre récipient collecteur (10) sur lequel la vanne (4) peut être renversée.

10. Dispositif de prélèvement d'échantillon selon la revendication 8 ou 9, caractérisé en ce que l'autre récipient collecteur (10) est logé dans le premier récipient collecteur (9).

**Claims**

1. A method of preparing a milk sample representative of a volume of milk, wherein during the transference of the milk from a first to a second container a component flow of proportional quantity from the main flow is branched off from the first container to the second container and collected, and on completion of the transference of the collected milk a component volume is taken as a sample, characterized in that the branching-off is performed in two phases with different proportionality factors, the proportionality factor in the first phase, referred to the total volume of milk to be transferred, being so high that at the end of this phase a given first component volume is achieved, which is then separated except for a residual volume corresponding to a substantially lower proportionality factor, while in the following, second phase the component flow is branched off with said substantially lower proportionality factor and the sample is taken after the residual volume has been mixed with the component volume branched off in the second phase.

2. A method according to Claim 1, characterized in that introduced between the two phases of the transference operation is a pause during which the component volume branched off in the first phase is separated except for the residual volume from the collecting container.

3. A method according to Claim 1, characterized in that with a continuous transference operation, during the separation of the milk not required from the first component volume, the milk deposited with the lower proportionality factor at the start of the second phase is collected and added to the residual volume before the sample is taken.

4. A sampling device on a milk collecting installation having a main pipe (2) which extends to a container (6) and from which a branch pipe (7, 8) having a valve (5) adjustable to different proportionality ratios extends to a collecting container (9) for samples which has an outlet (23) for the discharge of a sample, a return line controlled by a valve (15), and a level sensor (22) of a control device by which sampling can be controlled via valves (5, 15, 19, 17, 117), characterized in that when a predetermined milk level determined by the level sensor (22) is reached, the control device switches over the valve (5) adjustable to different proportionality ratios from a high proportionality ratio to a low proportionality ratio, and interrupts the further supply of milk branched-off, with a low proportionality ratio to the collecting container (9) until said collecting container (9) is emptied via the valve-controlled return line (16, 20) except for a residual volume corresponding to the low proportionality ratio.

5. A sampling device according to Claim 4, characterized in that the collecting container (9) for the separation of the milk of the first component volume not required for the sample has an overflow (17; 117) closeable by a valve (19; 15).

6. A sampling device according to Claim 5, characterized in that the overflow (17) is formed by a branch line (20) which is connected to the collecting container (9) and can be cut off by a valve (19).

7. A sampling device according to Claim 5, characterized in that the overflow (117) is formed by a tubular member which can be fitted by its lower opening on to the main outlet, which can be cut off by a valve (15), of the collecting container (9).

8. A sampling device according to one of Claims 4 to 7, characterized in that a further collecting container (10) is provided into which the milk can be delivered after the switching over of the valve (5) and whose contents can be transferred to the first collecting container (9) after its partial emptying.

9. A sampling device according to Claim 8, characterized in that extending from the valve (5) is a further branch line (8) which extends to the further collecting container (10) and to which the valve (4) can be switched over.

10. A sampling device according to Claims 8 or 9, characterized in that the further collecting container (10) is accommodated in the first collecting container (9).

Fig. 1

Fig. 2